# EUROPEAN PATENT SPECIFICATION

(11) **EP 1 027 375 B1**
(45) Date of publication and mention of the grant of the patent: **04.10.2006**
(21) Application number: 98952503.5
(22) Date of filing: 21.10.1998
(51) Int. Cl.: C07K 16/42, C12N 5/20, A61K 39/395

(54) **ANTI-IDIOTYPIC MONOCLONAL ANTIBODIES, THEIR USE IN THE ACTIVE IMMUNOTHERAPY OF MALIGNANT TUMORS, AND COMPOSITIONS CONTAINING THEM**
ANTI-IDIOTYPISCHE MONOKLONALE ANTIKÖRPER, DEREN VERWENDUNG ZUR AKTIVEN IMMUNTHERAPIE BÖSARTIGER TUMOREN, UND ZUSAMMENSETZUNGEN DIE DIESE ENTHALTEN
ANTICORPS MONOCLONAUX ANTI-IDIOTYPES, LEUR UTILISATION DANS L'IMMUNOTHERAPIE ACTIVE DE TUMEURS MALIGNES ET COMPOSITIONS LES RENFERMANT

(30) Priority: 21.10.1997 CU 11997
(43) Date of publication of application: 16.08.2000
(73) Proprietor: Centro de Inmunologia Molecular, Ciudad Habana 12100 (CU)
(72) Inventor: VASQUEZ LOPEZ, Ana Maria, Ciudad de la Habana (CU); PEREZ RODRIGUEZ, Rolando, Ciudad de la Habana 10500 (CU); IGLESIAS SIERRA, Eladio, Ciudad de laHabana 11700 C (CU); PEREZ GONZALEZ, Alexis, Ciudad de la Habana 13100 (CU); BOMBINO LOPEZ, Gumersinda, Ciudad de la Habana 12100 (CU); BEAUSOLEIL DELGADO, Irene, Playa, Ciudad de la Habana 12100 (CU)
(74) Representative: Prins, Adrianus Willem
(86) International application number: PCT/CU1998/000007
(87) International publication number: WO 1999/020656

(56) References cited:
- EP-A- 0 657 471
- EP-A- 0 661 061
- WO-A-93/10221
- WO-A-97/19113
- WO-A-97/34635
- US-A- 5 653 977
- E. IGLESIAS ET AL.: "Ganglioside vaccines: Anti-idiotypic monoclonal antibodies as antigen surrogates." BIOTECNOLOGIA APLICADA, vol. 14, no. 1, 1997, page 50 XP002091466 La Habana, Cuba
- R. PEREZ ET AL.: "What do gangliosides show us about idiotypic networks?" BIOTECNOLOGIA APLICADA, vol. 14, no. 1, 1997, page 42 XP002091467 La Habana, Cuba
- A. VAZQUEZ ET AL.: "Generation of a murine monoclonal antibody specific for N-glycolylneuraminic acid-containing gangliosides that also recognizes sulfated glycolipids." HYBRIDOMA, vol. 14, no. 6, December 1995, pages 551-556, XP002091468 New York, NY, USA cited in the application

## Description

### Technical Field

The present invention relates generally to anti-idiotypic monoclonal antibodies and their use as immunomodulators. More particularly, the present invention involves a murine monoclonal antibody, which was developed against a murine monoclonal antibody reactive with N-glycolyl containing gangliosides and with antigens expressed in cancer cells; and its inhibitory effect on tumor growth.

### Background

One of the strategies for the treatment of cancer has been the use of active immunotherapy, a treatment modality that has the objective of activate the natural potential of the immune system of the host against the tumor.

Since Niels Jerne in 1974 (Jerne, N. K. 1974. Ann Immunol 125C, 373-389) proposed his idiotypic network theory, new possibilities were opened in the study of effective therapies against cancer. Jerne's theory presented, for the first time, the immune system as a network of antibodies which can interact among them and with a large number of natural epitopes, through their variable regions or idiotypes (Id).

This complex set of idiotype-anti-idiotype interactions operates to regulate immune responses to antigens. Those antibodies made in response to the original antigen, named

Ab1, become themselves antigens and elicit the production of a second set of antibodies, called anti-idiotype (anti-Id) antibodies or Ab2, which also may be regulated by other antibodies referred as anti-anti-idiotype (Abl' or Ab3).

The original theory of Jerne continue being reviewed, and it was reported that the result of the interaction of Ab2 with lymphocytes bearing Ab1 has not to be necessarily the suppression of the immune response, but may be the stimulation of this response. Moreover, Jerne limited his theory to B lymphocytes and antibodies, but now is clear that T cells play an important role in the regulation through the idiotypes of T-cell receptors (Teitelbaum, D. et al, 1984 J. Immunol 132,1282-1285; Zanetti, M. et al (1986) J. Immunol 137, 3140-3146; Powell, J. et al (1988) 140, 3266-3272; Baskin, J.G. et al (1990) J Immunol 145,202-208; Furuyama, A. et al (1992) Anticancer Res 12, 27-32; Raychaudhuri, S. et al. J. Immunol 131, 271-278; Raychaudhuri et al (1987) J. Immunol 139, 3902-3910; Durrant et al (1994) Cancer Res. 54, 4837-4840).

An idiotype is immunologically defined by reactivity with more than one anti-Id that recognizes an idiotypic determinant or idiotope within a given idiotype. Thus, as a particular Ab1 expresses multiple idiotopes, when this Ab1 was administered to syngeneic animals a heterogeneous population of anti-Id antibodies is obtained.

Classification of the anti-Id antibodies focuses on their binding within the antigen-binding site or to some other region of the idiotype. If the binding of the Ab2 with the Ab1 is inhibited by the relevant antigen and if the Ab2 is also capable of inducing an antibody response of the same specificity as the Ab1, it mimics the natural antigen and is classified as Ab2β; this type of Ab2 is referred to as internal image anti-Id and they can act as surrogate antigens. Anti-Id that are not inhibited by antigen are designated Ab2α, these Ab2 react with Ab1 idiotopes which are not structurally related with the antigen-binding site.

In 1984 Bona and Köhler proposed a third type of anti-Id antibodies (Ab2γ), which are inhibited by antigen because of steric interference, this type of anti-Id reacts with idiotopes structurally associated with the antigen-binding site, but they do not mimic the antigenic epitope recognized by the Ab1 (Bona and Köhler (1984) Anti-idiotypic antibodies and internal image. In Monoclonal and anti-idiotypic antibodies: Probes for receptor structure and function, Venter JC, Frasser CM, Lindstrom J (eds), NY, Alan R. Liss, pp 141-149, 1984).

Based in Jerne's theory it has been developed two main approaches in the searching of vaccines to a large number of antigens, included tumor-associated antigens. The first approach is based in the presentation of epitopes in a different molecular environment, using Ab2β. Vaccines containing this type of anti-Id antibodies have been able to induce protective responses against virus, bacteria and parasites (Kennedy et al (1986) 232, 220-223; McNamara et al (1985) Science 226, 1325-1326). Also, Ab2β have been used to induce immune responses to tumor-associated antigens and positive results have been obtained in animal models and in clinical trials (Raychauhuri et al (1986) J. Immunol 137, 1743-1749; Raychauhuri et al (1987) J. Immunol 139, 3902-3910; Bhattacharya-Chatterjee et al (1987) J. Immunol 139, 1354-1360; Bhattacharya-Chatterjee et al (1988) J. Immunol 141, 1398-1403; Herlyn, D. et al (1989) Intern. Rev. Immunol 4, 347-357; Chen, Z-J et al. Cell Imm. Immunother. Cancer (1990) 351-359; Herlyn, D. et al (1991) In Vivo 5, 615-624; Furuya et al (1992) Anticancer Res 12, 27-32; Mittelman, A. et al (1992) Proc. Natl. Acad. Sci. USA 89,466-470; Durrant; LG. et al (1994) Cancer Res. 54, 4837-4840; Mittelman, A. et al (1994) Cancer Res. 54, 415-421; Schmitt, H. et al (1994) Hybridoma 13, 389-396; Chakrobarty, M. et al (1995) J. Immunother. 18, 95-103; Chakrobarty, M. et al (1995) Cancer Res. 55, 1525-1530; Foon, K. A. et al (1995) Clin. Cancer Res. 1, 1285-1294; Herlyn, D. et al (1995) Hybridoma 14, 159-166; Sclebusch, H. et al (1995) Hybridoma 14, 167-174; Herlyn, D. et al (1996) Cancer Immunol. Immunother. 43,65-76). Nevertheless, it has been demonstrated that the β character of an Ab2 is not enough to predict the biological effect that could induce the Ab2 (Raychauhuri et al (1986) J. Immunol 137, 1743-1749; Raychauhuri et al (1987) J. Immunol 139, 231-278); Maruyama et al (1996) Int. J. Cancer 65, 547-553).

The second approach is based in the network manipulation through regulatory idiotopes, which are not related with antigen binding, but involve idiotopes shared with other antibodies or T cells. It has been accumulated evidences that these anti-Id antibodies are also able to produce immune responses and protective effects (Paul, W. E. and Bona, C. (1982) Immunology Today 3, 230-234; McNamara M. K. et al (1985) Science 226, 1325-1326; Köhler y cols (1992) Proc. 8th Inter. Cong Immunol. Budapest, pp. 619).

Gangliosides are glycosphingolipids that contain sialic acid and are expressed in the majority of mammalian cell membranes. Although these antigens are present in normal tissues, they can be found in larger quantities and expressed in a different organization and conformation on the surface of malignant cells (Hakomori, S. (1985), Cancer Res. 45, 2405-2415; Miraldi, F. (1989) Seminars in Nuclear Medicine XIX, 282-294; Hamilton et al (1993) Int J Cancer 53, 1-81).

Although gangliosides are useful targets for immune responses, their immunogenicity are extremely poor, due to their carbohydrate nature and their self-antigen condition (Livingston, P. et al, (1995) Seminars in Cancer Biology 6, 357-366).

The N-glycolyl variant of sialic acid is expressed in normal tissues of most mammals; but it is very difficult to detect it in normal tissues from humans (Watarai, S. et al (1995) J. Biochem 117, 1062-1069. In the other hand, the presence of these antigens has been reported in colon cancer, melanoma, retinoblastoma and breast cancer, among others (Higachi, H et al (1984) Jpn J Cancer Res (Gann) 75, 1025-1029; Higachi, H et al (1985) Cancer Res. 45, 3796-3802; Hirabayashi, I. Et al (1987) Jpn J Cancer Res (Gann) 78, 1614-1620; Higachi, H. et al (1988) Jpn J Cancer Res (Gann) 79, 952-956; Miyake, M. et al (1990) Cancer 65,499-505; Devine, P. L. et al (1991) Cancer Res. 51,5826-5386; Vázquez, A.M. et al (1995) Hybridoma 14,551-556; Marquina, G. et al (1996) Cancer Res. 56, 5165-5171).

Immunization with vaccines containing gangliosides has resulted in prolonged survival of melanoma patients who developed anti-ganglioside antibodies (Livingston, P. et al (1987) Proc Natl Acad Sci USA 84, 2911-2915; Livingston, P. et al (1989) Cancer Res 49, 7045-7050; Livingston, P. (1995) Immunological Reviews 145, 147-166). Nevertheless, the problems of antigen together with their poor immunogenicity have made the use of anti-Id antibodies an attractive alternative for active immunotherapy in this antigenic model. A murine anti-Id mAb (4C10) was generated against a human IgM mAb (L612) that recognizes GM3 on human melanoma. Sera of mice immunized with this anti-Id mAb coupled with keyhole limpet hemocyanin (KLH) reacted strongly with an antigen-positive melanoma cell line and with purified GM3 which suggested that this anti-Id mAb carrying the internal image of GM3 (Ab2β) may be an effective tool for active specific immunotherapy in patients with melanoma (Yamamoto, S. et al (1990) J. Natl. Cancer. Inst. 82, 1757-1760; Irie, R. F. Patent Number US 5,208,146). The VL and VH from this anti-Id mAb have been cloned, sequenced and expressed as a chimeric mouse/human IgG1 antibody (Hastings, A. et al (1992) Cancer Res 52, 1681-1686).

Also, an alpha-type anti-idiotype mAb was generated against the human mAb 1612 useful in immunodiagnostic procedures (Irie, R. F. Patent Number US 5,208,146).

mAb BEC-2, a murine anti-Id mAb raised against a mouse mAb recognizing GD3 ganglioside (mAbR24), can mimic GD3 and can induce antibodies against this ganglioside despite expression of GD3 on normal rabbit tissue (Chapman, P. B. And Houghton, A. N. (1991) 88, 186-192). The results of a pilot study showed that BEC-2 plus BCG adjuvant significantly increased survival of patients with small cell lung cancer (Scrip Magazine (1996) pp 56-59; 33^{rd} American Society of Clinical Oncology annual meeting (1997)).

Immunizing rats with a murine mAb specific against GD2 (3F8), were generated anti-Id mAbs which when were tested as immunogens in mice could stimulate antibodies that reacted with ganglioside GD3. It was suggested that these anti-Id mAbs might be useful in vaccine construction (Cheung, N-K.V. et al (1993) Int. J. Cancer 54, 499-505).

Also, a human anti-Id mAb was generated using peripheral blood mononuclear cells from a patient treated with the murine mAb 14G2 specific against GD2. The immunization of rabbits with this human anti-Id mAb induced anti-GD2 antibodies and a DTH response to antigen-positive tumor cells. It was suggested that this antibody could be potentially used as a human anti-Id vaccine in patients with malignant melanoma (Saleh, M. N. et al (1993) J. Immunol 151, 3390-3398).

EP-A-0 657 471 relates to anti ganglioside monoclonal antibodies and their use in the specific active immunotherapy of malignant tumors. In particular, said document discloses the generation of an anti-ganglioside antibody P3 specifically binding to NGcGM3 (the N-glycolyl neuraminic acid linked to the internal galactose of the ganglioside GM3) and anti-idiotypic antibodies raised against said anti-ganglioside antibody P3, which anti-idiotypic antibodies recognize the original antigen, NGcGM3.

In the article "Ganglioside vaccine: anti-idiotypic monoclonal antibodies as antigen surrogates (Iglesias, E., et al. Biotecnologia Aplicada, Vol. 14, No. 1, 1997, page 50, XP-002091466) are described seven IgG1 anti-idiotypic monoclonal antibodies (MAbs) which reacted strongly with P3 MAb and no reactivity was observed with the other anti-ganglioside IgM MAbs tested. The seven anti-idiotypic MAbs had the capacity to block P3 binding to NGcGM3 in a concentration range between 1 to 10 µg/ml. Five of these anti-idiotypic were capable to elicit a humoral response against NGcGM3, being classified as Ab2β and Ab2α anti-idiotypic monoclonal antibodies.

On the other hand, Pérez et al. ("What gangliosides show us about idiotypic networks". Biotecnologia Aplicada, Vol. 14, No. 1, 1997, page 42, XP-002091467) also describes that the anti-ganglioside antibody P3 raised strong IgG anti-idiotypic responses (Ab2, titers 1/10 000 to 1/50 000) in Balb/c mice. The most of these specific Ab2 clones obtained were able to block binding of P3 MAb to GM3 (NeuGc). These antibodies, as in the above-referred case, were able to induce a natural autoantibody response to gangliosides, being too classified as Ab2β anti-idiotypic antibodies.

As it is apparent from the above background, until now none gamma type anti-idiotype monoclonal antibody has been generated against monoclonal antibodies which recognize N-glycolyl containing-gangliosides, which at the same time, are capable to exert an anti-tumoral effect in an animal model.

### Disclosure of the Invention

The present invention relates generally to anti-idiotypic (anti-Id) monoclonal antibodies (mAb) and their use as immunomodulators for cancer treatment. More particularly, the invention involves a gamma type anti-idiotypic (Ab2γ) monoclonal antibody raised against a murine antibody against N-glycolyl-containing gangliosides produced by the hybridoma deposited under the accession number ECACC 94113026, the Ab2γ being obtained from the hybridoma cell line deposited under the accession number ECACC 97112901. Moreover, the invention involves said hybridoma deposited under the accession number ECACC 97112901, a pharmaceutical composition containing an effective amount of said anti-idiotypic monoclonal antibody, together with a diluent, an adjuvant or a transporting molecule; and the use of said monoclonal antibody for the manufacture of a medicament for treating malignant neoplasias.

In accordance with the present invention, an objective of this invention is to provide a new anti-Id mAb capable to induce a predominant anti-idiotypic response in xenogeneic models and to exert a protective effect in mice or other animal species bearing malignant tumors.

### Detailed description of the invention.

### IMMUNIZATION PROCEDURE FOR OBTAINING ANTI-IDIOTYPIC (Ab2) ANTIBODY RESPONSE TO ANTIGANGLIOSIDE mAbs

Mice and other mammalian species are immunized with 25-200 µg doses of purified anti-ganglioside mAbs with or without adjuvant and optionally coupled to a transporting protein.

Animals receive 2-6 doses of anti-ganglioside mAbs at 14 to 30 days intervals between doses. Possible immunization routes are intraperitoneal, subcutaneous, endovenous or a combination of these.

Before and during the immunization period animal blood serum samples are taken and Ab2 antibody level response is determined any known immunoassay methods. Animal serum dilutions are incubated with the anti-ganglioside mAb used as immunogen, or other anti-ganglioside mAbs not used in the immunization protocol.

Experiments were also performed to define the capacity that the serum of the immunized animals has for blocking the binding of the anti-ganglioside mAb used as immunogen to its antigen.

### ANTI-IDIOTYPIC mAb PRODUCTION AGAINST ANTI-GANGLIOSIDE mAbs.

Mice with high Ab2 antibody titers receive a re-immunization with the mAb used as immunogen three days before obtaining the antibody producing cells. Spleen cells should be preferentially used although; other antibody producing cells may be selected.

These cells are fused with myeloma cells that give the hybrid cells or hybridomas the property of **in vitro** and **in vivo** reproduction. Cellular fusion may be performed by any of the known methods.

The antibodies produced by the hybridomas are tested by immunoassay methods, preferably by an immuno-enzymatic assay in which hybridoma supernatants are incubated with the mAb used as immunogen and with other anti-ganglioside mAbs not used in the immunizations.

The capacity of the hybridoma supernatant of blocking the binding of the anti-ganglioside mAb used as immunogen to its antigen is determined by incubating the supernatants with adequate dilutions of the anti-ganglioside mAb followed by incubation of said antibody with its antigen.

The selected hybridomas are cloned at least twice and the resultant mAbs are produced **in vitro** and **in vivo** as described above.

The anti-idiotypic mAbs obtained recognize the anti-ganglioside mAbs and can posses the capacity of blocking the binding of the anti-ganglioside mAb to its antigen.

### IMMUNIZATION PROCEDURE FOR OBTAINING ANTI-ANTI-IDIOTYPIC (Ab3) ANTIBODY RESPONSE TO Ab2 mAbs IN XENOGENEIC MODELS.

Monkeys or other xenogeneic specie are immunized with anti-idiotypic mAbs. These mAbs can be administered with or without adjuvant and optionally coupled to a transporting protein before being used as immunogen.

Each animal received from 2 to 8 doses of 250 µg to 2 mg of the anti-idiotypic mAb at time intervals of 7 to 30 days between doses.

Immunization routes can be intradermal, subcutaneous, endoveneous, intraperitoneal or a combination of these.

Animal blood samples are obtained before and during the immunization protocol and the presence of Ab3 antibody response are monitored using any of the known immunoassay methods.

The administration to animals of anti-idiotypic mAbs, produced by the immunization with anti-ganglioside mAbs, can induce a predominant antibody response against the idiotype of the anti-idiotypic mAb used as immunogen, without the induction of antigen-specific antibody response.

### ANTI-TUMORAL EFFECT OF ANTI-IDIOTYPIC mAbs TREATMENT

Anti-idiotypic mAbs are administered in an effective quantity with or without adjuvants and optionally coupled to a transporting protein before being used. The term effective quantity should be understood as meaning an amount of the anti-idiotypic mAb required to achieve an anti-tumoral effect. The treatment with the anti-idiotypic mAbs is done prior or after the experimental administration of malignant cells in animals.

Animals receive 1-5 doses of 10 -200 µg of the anti-idiotypic mAbs, at time intervals of 7-30 days between doses.

Immunization routes can be intradermal, intraperitoneal, subcutaneous, endoveneous or a combination of these.

After the treatment with the anti-idiotypic mAbs, the tumor incidence and the survival in the group treated with the anti-idiotypic mAbs can be compared to control groups, otherwise treated and maintained in the same manner.

Anti-idiotypic mAb treatment significantly increased survival of animals bearing tumors and diminished lung metastases.

### Example 1: Generation of an anti-idiotypic antibody against P3 mAb in a syngeneic model.

Female Balb/c mice, 6-8 weeks old, were immunized with two doses of 50 µg of purified P3 mAb (anti-N-glycolil containing-gangliosides (EACC accession number 94113026, European Patent Application EP 657471 A1; Vázquez, AM. et al, Hybridoma (1995) 14,551-556) coupled to KLH, at 14 days intervals between doses.

Three days after the last immunization, spleens of mice with high serum levels of Ab2 antibodies against P3 mAb were removed and a cellular suspension prepared by pressing the tissue through a stainless steel sieve or perfusing the spleen.

Fusion was performed by the method described by Köhler and Milstein (1975, Nature (Lond) 256, 495-497), with slight modifications.

Murine spleens cells were fused with the cells of the non-secreting murine myeloma P3/X63 Ag8 6.5.3. obtainable from ECACC, No. 85011420, in a ratio 10:1, in 0.5 ml of fusion medium containing 42% polyethylene glycol in RPM!-1640 medium.

After fusion cells were cultivated in a HAT (hypoxanthine-aminopterin and thymidine) selective medium at 37°C in a 5% CO2 humid atmosphere.

Ten to fifteen days after fusion evaluation of presence of antibodies in hybridoma supernatants was determined by ELISA. ELISA plates (high binding COSTAR) were incubated overnight at 4°C with 10 µg/ml of P3 mAb and other IgM anti-ganglioside mAb, in carbonate-bicarbonate buffer pH 9.8.

Plates, after washing with PBS containing 0.05% Tween 20, were blocked with the same buffer containing 1% BSA during one hour at 37°C.

Washing step was repeated and 50 µl/well of the supernatants were added. After incubating for 2 hours the plates were washed again an alkaline phosphatase goat anti-murine IgG Fc region conjugate antiserum was added. After washing, 100 µl/well of the substrate solution (1 mg/ml of p-nitrophenylphosphate diluted in diethanolamine buffer, pH 9.8) was added. Absorbance was measured at 405 nm in an ELISA reader.

Also, the capacity of the supernatants to block the binding of P3 mAb to NeuGcGM3 was determined by an indirect ELISA performed on polyvinyl chloride activated plates (ICN-FLOW) with immobilized NeuGcGM3, according to the following method: Fifty microliters of the ganglioside in methanol (4 µg/ml) were added to each well. Methanol was evaporated by placing the plates at 37°C during one hour. Later, 150 µl/well of buffer TRIS-HCl 0.05 M pH 7.8 containing 1% BSA were added and plates were incubated at 37°C for 30 minutes.

Hybridoma supernatants were incubated with 4 µg/ml of P3 mAb for 3 hours at 37°C and then, 50 µl/well of the mixtures were added and the plates were incubated at 37°C for 90 minutes.

Wells were washed 4 times with 200 µl of PBS and 50 µl of alkaline phosphatase anti-mouse immunoglobulins conjugate antiserum, adequately diluted were added. After washing with PBS the wells were incubated with the substrate solution as described previously.

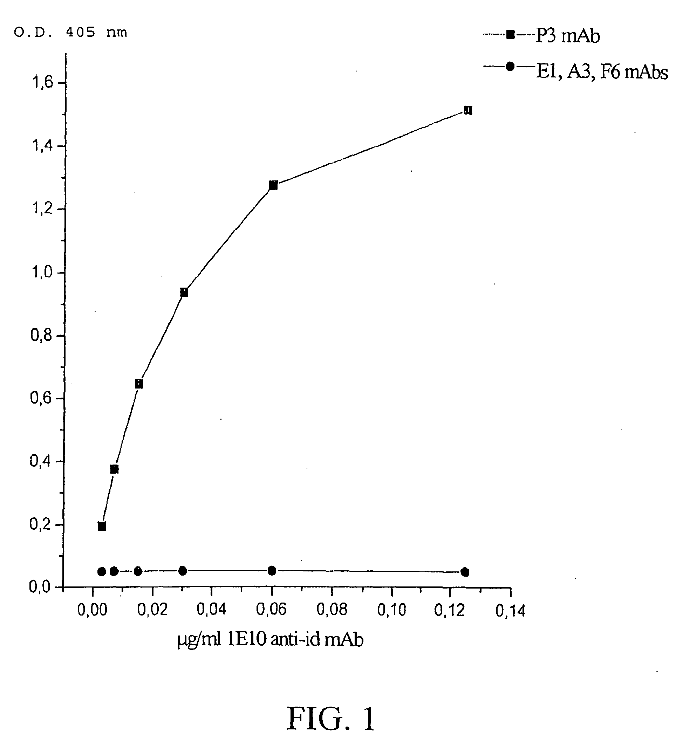

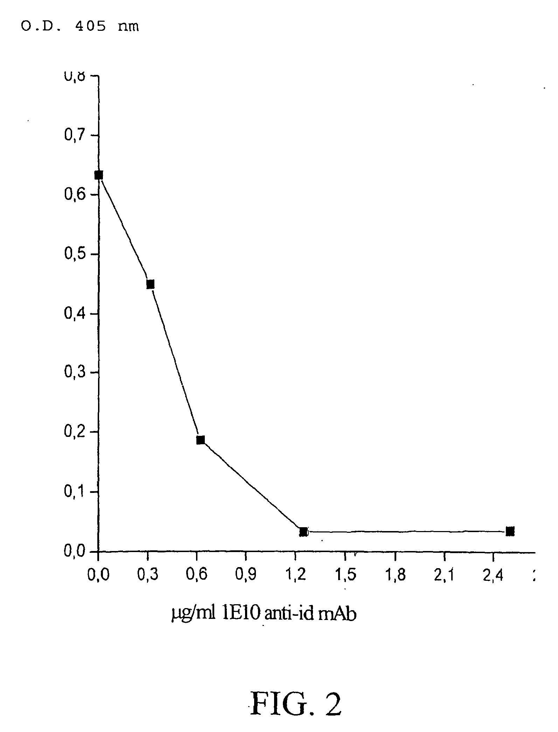

An anti-idiotypic mAb, named 1E10, of the IgG1 subclass was obtained. This 1E10 anti-IdP3 mAb was very specific to the P3 mAb used as immunogen. It does not react with other anti-ganglioside antibodies, such as E1, A3 and F6 (Figure 1). This anti-idiotypic mAb was able to inhibit the binding of the P3 mAb to NeuGcGM3 (Figure 2) and to a P3 positive breast tumor cell line.

### Example 2: Generation of anti-anti-idiotypic (Ab3) antibody response to 1E10 anti-Id mAb in a xenogeneic model.

Aluminum hydroxide-precipitated 1E10 anti-IdP3 mAb was used to immunize intradermally Cynomolgus monkeys at .14 days intervals with a dose of 2 mg of mAb per injection. The animals received several doses of the mAb.

Serum samples were taken before and after the immunizations. The presence of Ab3 antibody response in monkey serum was determined by ELISA. ELISA plates (high binding Costar) were incubated overnight at 4°C with 10 µg/ml of 1E10 mAb or its F(ab')2 fragments in carbonate-bicarbonate buffer, pH 9.8.

Plates, after washing with PBS containing 0.05% Tween 20, were blocked with the same buffer containing 1% BSA during one hour at 37°C.

Washing step was repeated and 50 µl/well of the different serum dilutions were added. After incubating for 2 hours at 37°C, the plates were washed again and alkaline phosphatase goat anti-human immunoglobulin, anti-IgM or anti-IgG conjugate antisera were added. After washing, the substrate solution was added as previously described.

Blocking experiments were performed to distinguish between the anti-isotypic and anti-idiotypic antibody responses.

Monkey serum was incubated overnight at 4°C with 500 µg/ml of an mAb with the same isotype as 1E10 mAb, but with different specificity. Then, the remnant serum reactivity against 1E10 mAb was measured by ELISA, as described above.

The monkey sera were checked for their ability to inhibit the binding of biotinilated P3 mAb to 1E10 mAb F(ab')2 fragments coated onto ELISA plates using the ELISA method described above, but where after incubating the coated plates with the serum dilutions, 50 µl/well of biotinilated P3 mAb were added. After an incubation for one hour at 37°C, plates were washed and 50 µl/well of avidin-biotin-peroxidase complex were added for one hour at 37°C. After washing, the substrate buffer (8 mg of o-phenylenediamine in 12 ml of phosphate-citrate buffer, pH 5.0) was added. Absorbance was measured at 492 nm in an ELISA reader.

Sera from monkeys immunized with 1E10 anti-IdP3 mAb reacted strongly with 1E10 F(ab') 2 fragments (Fig. 3). Sera from immunized monkeys bound specifically to the immunized 1E10 mAb, with less reactivity with unrelated mAb (ior C5). The presence of antibodies against 1E10 idiotype were confirmed after incubated animal sera with the irrelevant, mAb, due to an strong reactivity of the adsorbed sera with the 1E10 anti-IdP3 mAb; surprisely, the anti-idiotypic antibody response induced was superior in comparison to the anti-isotypic antibody response generated (Fig. 4). Monkey Ab3 sera also inhibited the binding of biotinilated P3 mAb to 1E10 anti-Id mAb, indicating that monkey Ab3 sera share idiotopes with P3 mAb (Fig. 5). A predominant IgG antibody response was generated against 1E10 anti-IdP3 mAb (Fig 6). Sera from the monkeys reacted with 1E10 F(ab')2 fragments even 4 months after the animals received the last immunization (Fig 7), Ab3 antibodies with inhibitory capacity of P3 mAb binding to 1E10 were also detected at that time. No antibodies against NeuGcGM3 were detected in animal sera.

It is demonstrated that 1E10 anti-idiotypic mAb is a gamma-type, since 1E10 anti-idiotypic mAb is capable to inhibit the binding of P3 mAb with its antigen and it is not able to induce the production of P3-like antibodies in the animals.

### Example 3: Treatment of mice with 1E10 anti-Id mAb.

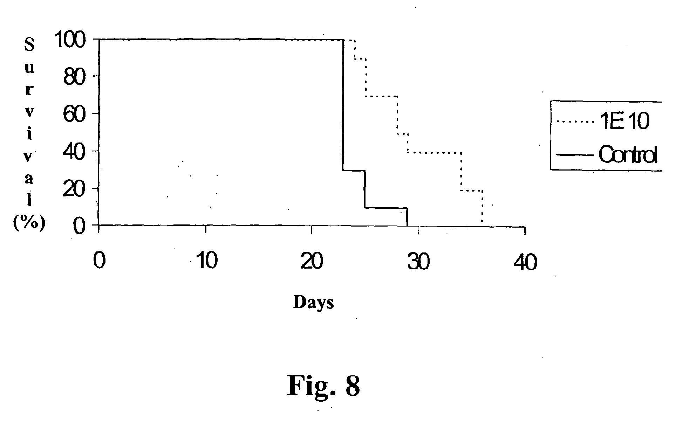

C57BL/6 mice were immunized with five doses of 50 µg of aluminum hydroxide-precipitated 1E10 anti-IdP3 mAb at 14 days intervals. On week later, mice were injected subcutaneously with 5 x 10³ B16 melanoma cells. Animals treated in the same way, but which did not receive the mAb were used as controls. Kaplan-Meyer survival curves are shown in Figure 8; survival was significantly better in the group immunized with 1E10 anti-IdP3 mAb than in the control group.

C57BL/6 mice were inoculated intravenously with 50 x 10³ Lewis lung cancer cell. Fourteen days later, 10 µg of 1E10 was administered intravenously and after 6 days, mice were sacrificed and the number of lung metastases was counted. Animals treated with an irrelevant IgG mAb or which only received PBS were used as controls. Seven of the 10 animals treated with 1E10 anti-P3mAb did not developed lung metastases, in the other 3 animals the maximum total number of metastases were 3. In contrast, all the animals treated with the irrelevant IgG1 or those which only received PBS developed lung metastases. These results indicate that the treatment with this gamma-type anti-Id mAb had a protective effect against tumors.

### BRIEF DESCRIPTION OF THE DRAWINGS:

Figure 1: shows the reactivity of 1E10 anti-IdP3 mAb against P3, E1, A3 and F6 anti-ganglioside mAbs.
Figure 2: shows the results of an inhibition assay where the P3 mAb was incubated with the 1E10 anti-IdP3 mAb, and later the reactivity of P3 mAb to NeuGcGM3 were measured by ELISA.

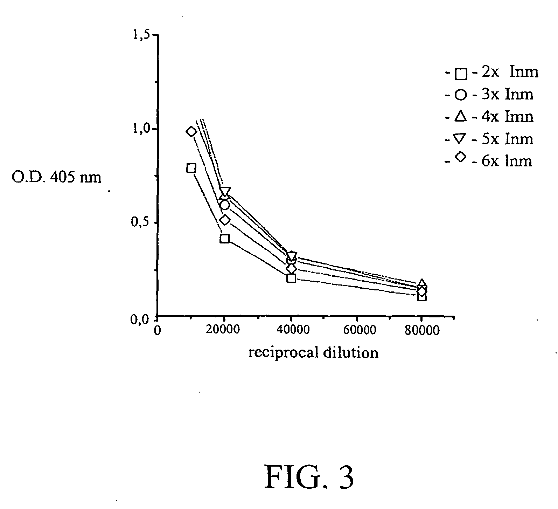

Figure 3: Shows the reactivity of monkey serum antibodies with 1E10 F(ab')2 fragments measured by ELISA after the animal received different doses of the aluminum hydroxide-precipitated 1E10 anti-Id mAb.

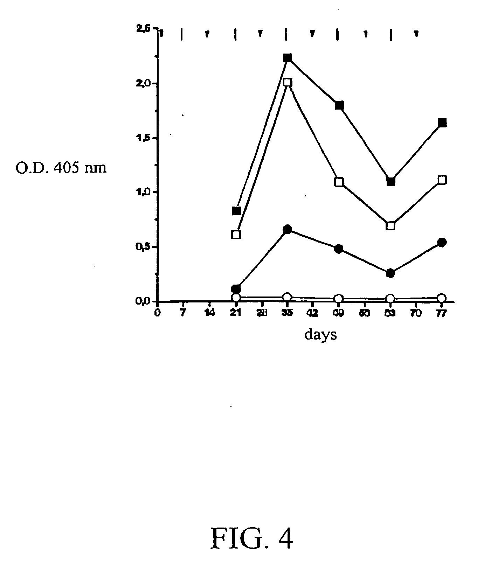

Figure 4: Shows the results of an inhibition assay, where both reactivities, of a monkey non-pre-adsorbed serum with 1E10 mAb (-■-) and of a monkey non-pre-adsorbed serum with isotype matched irrelevant ior C5 mAb (-•-), were measured by ELISA, as well as the binding of serum antibodies to 1E10 mAb (-□-) and to the irrelevant ior C5 mAb (-O-). Arrows indicate time of immunization and continue lines time of bled.

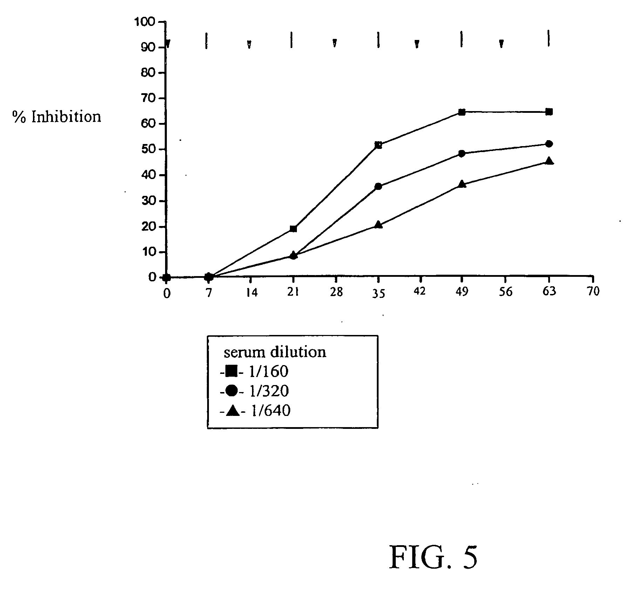

Figure 5: shows the inhibition of P3 mAb binding to 1E10 anti-Id mAb by the serum of a monkey immunized with 1E10 mAb, measured by ELISA. Arrows indicate time of immunization and continue lines time of bled.

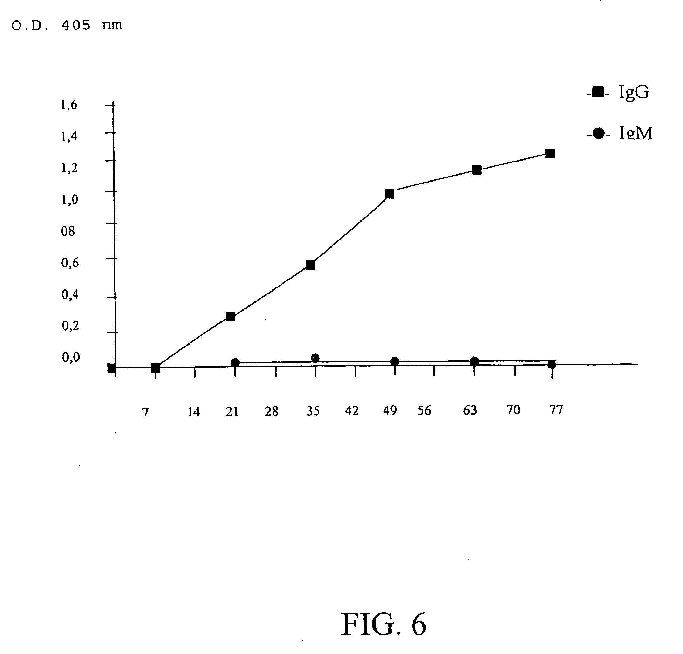

Figure 6: shows the kinetic of IgM and IgG antibody response against 1E10 mAb in the serum of a monkey immunized with the anti-idiotypic mAb, measured by ELISA. Arrows indicate time of immunization and continue lines time of bled.

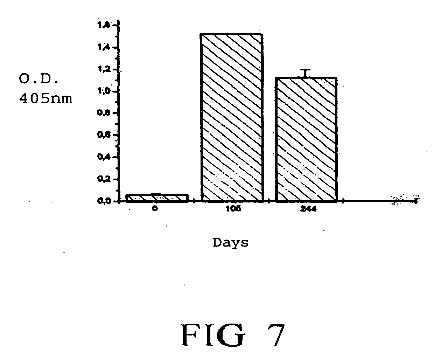

Figure 7: shows the recognition of 1E10 F(ab')2 fragments by monkey pre-immune serum; by the serum obtained after the monkey received the last dose of aluminum hydroxide-precipitated 1E10 anti-idiotypic mAb (indicated with the arrow), and by the serum obtained 4 months after the animal received the last immunization.
Figure 8: shows Kaplan-Meyer curves for survival in mice treated with 1E10 anti-IdP3 mAb and inoculated with B16 melanoma cells.

## Claims

1. A gamma type anti-idiotypic (Ab2γ) monoclonal antibody raised against a murine antibody against N-glycolyl-containing gangliosides produced by the hybridoma deposited under the accession number ECACC 94113026, the Ab2γ being obtained from the hybridoma cell line deposited under the accession number ECACC 97112901.

2. A hybridoma producing the monoclonal antibody of claim 1, deposited under the accession number ECACC 97112901.

3. A pharmaceutical composition containing an effective amount of the anti-idiotypic monoclonal antibody of claim 1, together with a diluent, an adjuvant or a transporting molecule.

4. Use of the monoclonal antibody of claim 1 for the manufacture of a medicament for treating malignant neoplasias.

## Patentansprüche

1. Ein anti-idiotypischer (Ab2γ) monoklonaler Antikörper vom Gammatyp, der gerichtet ist gegen einen murinen Antikörper gegen N-Glykolyl-haltige Ganglioside, der erzeugt wird durch das unter der Zugangsnummer ECACC 94113026 hinterlegte Hybridoma, wobei der Ab2γ erhalten wird aus der unter der Zugangsnummer ECACC 97112901 hinterlegten Hybridomazelllinie.

2. Hybridoma, das den monoklonalen Antikörper nach Anspruch 1 erzeugt, das unter der Zugangsnummer ECACC 97112901 hinterlegt ist.

3. Pharmazeutische Zusammensetzung, die eine wirksame Menge des antiidiotypischen monoklonalen Antikörpers nach Anspruch 1 enthält, zusammen mit einem Verdünnungsmittel, einem Hilfsmittel oder einem Transportmolekül.

4. Verwendung des monoklonalen Antikörpers nach Anspruch 1 zur Herstellung eines Medikaments zur Behandlung von bösartigen Neoplasien.

## Revendications

1. Anticorps monoclonal anti-idiotypique de type γ (Ab2γ) dirigé à l'encontre d'un anticorps murin dirigé contre des gangliosides contenant des N-glycolyle produit par l'hybridome déposé sous le numéro d'accès ECACC 94113026, ledit anticorps Ab2γ étant obtenu à partir d'une lignée cellulaire hybridome déposée sous le numéro d'accès ECACC 97112901.

2. Hybridome produisant l'anticorps monoclonal selon la revendication 1, déposé sous le numéro d'accès ECACC 97112901.

3. Composition pharmaceutique contenant une quantité efficace de l'anticorps monoclonal anti-idiotypique selon la revendication 1 avec un diluant, un adjuvant ou une molécule transporteur.

4. Utilisation de l'anticorps monoclonal selon la revendication 1 pour la fabrication d'un médicament pour le traitement d'une néoplasie maligne.
